(19) Europäisches Patentamt
European Patent Office
Office européen des brevets

(11) Publication number: **0 004 732**
**B1**

(12) # EUROPEAN PATENT SPECIFICATION

(45) Date of publication of patent specification: **23.12.81**

(21) Application number: **79300458.1**

(22) Date of filing: **22.03.79**

(51) Int. Cl.³: **C 07 C 27/00,**
C 07 C 33/22,
C 07 C 69/14,
C 07 C 67/00, C 07 C 29/14

(54) **Preparation of 2-phenylethanol and 2-phenylethyl acetate.**

(30) Priority: **04.04.78 US 893452**
**04.04.78 US 893502**

(43) Date of publication of application:
**17.10.79 Bulletin 79/21**

(45) Publication of the grant of the European patent:
**23.12.81 Bulletin 81/51**

(84) Designated Contracting States:
**BE DE FR GB IT LU NL SE**

(56) References cited:
**FR - A - 1 034 619**
**FR - A - 2 359 811**
**US - A - 3 285 948**

(73) Proprietor: **CHEM SYSTEMS, Inc.**
**747 Third Avenue**
**New York New York 10017 (US)**

(72) Inventor: **Sherwin, Martin Barry**
**90 Ashburn Road Wayne**
**Passaic New Jersey 07470 (US)**
Inventor: **Brownstein, Arthur Marvin**
**432 George Place Wyckoff**
**Bergen New Jersey 07481 (US)**
Inventor: **Peress, Jimmy**
**15 Alexander Drive**
**West Haven New Haven Connecticut 06516 (US)**

(74) Representative: **Harrison, Michael Robert et al,**
**Urquhart-Dykes & Lord Tower House Merrion Way**
**Leeds, LS2 8PB (GB)**

**EP 0 004 732 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Preparation of 2-phenylethanol and 2-phenylethyl acetate

It has long been desirable to prepare β-phenylethyl alcohol and β-phenylethyl acetate in a low cost process. The former is a valuable intermediate in the preparation of fragrances, and both are useful for making styrene, a commercial chemical with widely varying uses. In the past, it has been proposed that β-phenylethyl alcohol be prepared from benzyl alcohol. For example, such a preparation, in the presence of a cobalt catalyst, has been described by Wender, I. et al., *J. Am. Chem. Soc.* 71 (1949), pages 4160—4161. This early work is summarized by Orchin in *Advances in Catalysis,* Vol. V (1953), pages 393—414. Orchin reports that, at 185°C., a 50—60% yield of toluene and a 25—35% yield of β-phenylethyl alcohol is obtained. Other workers have also experimented with this reaction, particularly Y. B. Kryukov et al, who describe a vapor phase reaction over an iron, alumina, vanadium and potassium catalyst at 450°C. and a pressure of 5 × $10^6$ Pa (50 atmospheres) in *Neftekhimiya,* 1970, 10 (1), at page 83.

Though not related to the formation of β-phenylethyl alcohol, homologation has been described by a series of patents assigned to Commercial Solvents, including U.S. Patents 3,248,432 and 3,285,948, British Patent 951,506, and Belgian Patents 618,413 and 625,939. These references are primarily concerned with the homologation of methanol to form ethanol.

Until now, it was believed that benzyl alcohol of a high purity was required as the feedstock for the homologation step. Such feedstocks, while obtainable, can only be obtained after complicated purification schemes because benzyl alcohol, prepared by conventional processes, contains substantial quantities of other related oxygenated products. For example, benzyl alcohol may be prepared from toluene by two processes.

In the first process, the toluene is acetoxylated by reaction of molecular oxygen in the presence of acetic acid to form benzyl acetate and the benzyl acetate is then hydrolyzed to form benzyl alcohol. However, high purity benzyl alcohol is not obtained from this hydrolysis because the acetoxylation reaction yields benzaldehyde and benzylidene diacetate as by-products. When these by-products are added to the hydrolysis step along with the benzyl acetate, the benzylidene diacetate is converted to additional amounts of benzaldehyde. Furthermore, because the hydrolysis of benzyl acetate is an equilibrium reaction, the effluent of the hydrolysis contains a significant amount of benzyl acetate. In other words, by following this first process, benzyl alcohol is formed with both benzyl acetate and benzaldehyde by-products.

In the second process, the toluene is first oxidized to benzaldehyde and the benzaldehyde is thereafter reduced to benzyl alcohol. Unless a costly finishing step is employed, a significant amount of benzaldehyde, approximately 3% or more, remains in the product effluent.

Heretofore, the elimination of benzaldehyde and benzyl alcohol was deemed necessary because such compounds cannot *per se* be homologated to β-phenylethyl alcohol or β-phenylethyl acetate in any substantial quantities.

Furthermore, the product from the homologation reaction was treated to recover the B-phenylethyl alcohol and β-phenylethyl acetate by first permitting the effluent to settle to form an organic phase and an aqueous phase. The organic phase was then separated, and processed to remove low boiling compounds. Thereafter, generally in a subsequent distillation, the β-phenylethyl alcohol and the β-phenylethyl acetate were separated as a distillate to obtain a high purity product. The residue from this latter distillation containing substantial amounts of dibenzyl ethers. These materials were considered undesirable by-products and represented a debit to the economics of the overall process.

In the first embodiment of the present invention, it has surprisingly been found that improved yields of β-phenylethyl alcohol and β-phenylethyl acetate can be obtained by the homologation of benzyl alcohol, benzyl acetate, and benzaldehyde. This discovery is of substantial economic importance, firstly, because it permits the homologation to proceed without the necessity of removing either of the foregoing oxygenated benzyl compounds, compounds which frequently appear as co-products in the preparation of benzyl alcohol, from the feed stream; and, secondly, because it enhances the overall selectivity of the process by allowing such materials, which often appear in the homologation effluent, to be recycled.

The second embodiment of the subject invention concerns a process for improving the yields of β-phenylethyl alcohol and β-phenylethyl acetate obtained in the homologation of benzyl alcohol by the recycling of the high boiling dibenzyl ether by-products. This discovery is of substantial economic importance because heretofore these dibenzyl ethers were regarded as undesirable by-products of the reaction. Additionally, the finding that such materials can be advantageously recycled simplifies the catalyst recovery, particularly where a promoted cobalt catalyst is utilized.

The first embodiment of the present invention relates to the preparation of β-phenylethyl alcohol and β-phenylethyl acetate by the homologation of benzyl alcohol. This embodiment also relates to such a process wherein benzaldehyde, benzyl acetate, or both, are included in the feedstream to the homologation reaction. This embodiment of present invention also relates to various combinations of process steps for preparing β-phenylethyl alcohol and β-phenylethyl acetate from precursors of benzyl alcohol.

One combination of process steps of this embodiment comprises the hydrolysis of benzyl acetate

to benzyl alcohol followed by homologation of the organic phase from the hydrolysis to form the β-phenylethyl alcohol. In view of the discovery that the homologation can take place in the presence of residual benzyl acetate, it is unnecessary to further purify the organic phase from the hydrolysis reaction and the degree of conversion of benzyl acetate to benzyl alcohol is less critical. In other words, it is possible to use a simple hydrolysis step without the necessity of encumbering the reaction with large amounts of water or unduly long reaction periods so as to eliminate or avoid the presence of substantially all of the benzyl acetate in the hydrolysis mixture.

It is well known that toluene may be acetoxylated in the presence of acetic acid and oxygen to form high yields of benzyl acetate and minor amounts of benzaldehyde and benzylidene diacetate as by-products. In a second combination of process steps of this embodiment, this acetoxylation of toluene may be followed by hydrolysis and homologation, and the by-products of the acetoxylation need not be separated since they may be passed directly to both the hydrolysis reactor and the homologation step. As noted above, the benzaldehyde as well as the benzyl acetate may be homologated to β-phenylethyl alcohol and β-phenylethyl acetate in the presence of benzyl alcohol.

A further combination of process steps of this first embodiment of the present invention relates to the reduction of benzaldehyde followed by homologation. In this process, the benzaldehyde is reduced in the presence of hydrogen and the reaction product is passed directly to the homologation reaction. Where a one-step reduction is performed in the conventional manner, the effluent contains a small amount of residual unconverted benzaldehyde, i.e., from 3 to 15% based on total benzyl compounds. Conventionally, the residual benzaldehyde is reduced in a second "finishing" step. However, in accordance with the present invention, the benzaldehyde and benzyl alcohol may be fed directly to the homologation step and the need for the finishing step is eliminated.

A still further combination of process steps of this embodiment relates to subjecting benzaldehyde, formed by the oxidation of toluene, to reduction and homologation. Accordingly, it will be seen that β-phenylethyl alcohol may be prepared from toluene by two distinct combinations of reaction steps, both starting from the readily available raw material, toluene.

In the homologation reaction of one embodiment of the present invention, the feedstock must contain at least 25 mole % benzyl alcohol, preferably at least 50 mole %. From 3 to 15 mole % of benzaldehyde, preferably 3 to 10 mole %, and from 5 to 75% of benzyl acetate, preferably from 5 to 50 mole %, are present. These percentages are based on the total amount of benzyl alcohol, benzaldehyde and benzyl acetate (i.e., benzyl compounds) in the feedstock.

In addition, the feedstock may also contain trace amounts of other components and advantageously 0.1 to 20 weight % of water based on the total amount of the feedstock. Preferably, from 1 to 10% of water is present. In determining the amount of water to be used, it is desirable to avoid the formation of a separate water phase. A water phase is detrimental because the catalyst is water-soluble and will be extracted from the organic reaction phase by a water phase. Greater amounts of water may be used if a coupling solvent which prevents the formation of a separate water phase is added to the reaction medium.

While a feedstock for the reaction may be obtained from the reduction and hydrolysis steps herein described, a useful feedstock may also be obtained by combining a benzyl alcohol feed with an organic recycle stream rich in benzyl acetate.

Any homologation catalyst may be used in the reaction. Most of such catalysts that are described in the foregoing references are cobalt or cobalt-promoted catalysts. These are generally present in amounts from 0.25% to 5%, calculated as moles of cobalt catalyst (as Co) to moles of benzyl compounds multiplied by 100. Over this range, variations in the amounts of catalyst are not particularly critical. As practical matter, the amount of catalyst employed is from 1 to 3 mole %. The cobalt catalyst added to the system is selected so as to be soluble in the reaction medium. The active form of the cobalt catalyst is believed to be cobalt tetracarbonyl hydride [$HCo(CO)_4$]. This cobalt compound can be formed *in situ* by adding to the system a cobalt tetracarbonyl hydride-yielding compound, such as an organic salt of cobalt, particularly a water-soluble compound, e.g., cobalt acetate, cobalt formate, or cobalt propionate. Such materials are readily converted to the active cobalt form during the reaction.

Promoters may be employed in the reaction; these include a ruthenium source and an iodide source, used either alone or in combination. A ruthenium source is most desirably added as the halide, and from 0.02 to 0.30 atoms of ruthenium, preferably from 0.04 to 0.15 atoms of ruthenium, should be present for each atom of cobalt. Ruthenium may be introduced as a metallo-organic chemical. The iodide source may be added to the reaction system as an iodo-organic compound, such as benzyl iodide or hydrogen iodide, or as a salt form of the iodide, such as an alkali metal iodide. Generally, from 0.05 to 2.0 atoms of iodine, preferably from 0.10 to 1.0 atoms of iodine, per atom of cobalt should be present. If ruthenium is present when an iodo-organic compound is added to the reaction system, a metallo-organic compound may be formed. Promoted catalysts may be prepared in accordance with the teaching of U.S. Patent 3,285,948, assigned to Commercial Solvents.

The amount of hydrogen and carbon monoxide added in the reaction is generally in stoichiometric excess of the amount of benzyl alcohol used. As a minimum, at least stoichiometric quantities must be added and excesses up to ten times the stoichiometric amount are useful. As little as one-half mole of hydrogen to each mole of carbon monoxide may be used, and up to five moles of hydrogen to carbon

monoxide may be used. The most preferred range is from 3:1 to 1:1. Sufficient carbon monoxide must be present to maintain the cobalt catalyst in its active state.

Reaction temperatures may be from 100 to 165°C., preferably from 120 to 150°C. The reaction pressure should be from at least $6.8 \times 10^6$ Pa (70 atmospheres), preferably from $6.8 \times 10^6$ to $4.9 \times 10^7$ Pa (70 to 500 atm.), most preferably from $1.9 \times 10^7$ to $3.9 \times 10^7$ Pa (200 to 400 atm.). Increased pressures tend to favor selectivity of the B-phenylethyl alcohol. However, the use of higher ranges of pressure is limited by practical considerations such as the selection of equipment and safety factors.

The reaction period is not critical, but should be selected so as to achieve acceptable conversions without unduly lengthening the process cycles. As a practical matter, the reaction period would range from one-half to four hours.

Where benzyl acetate is used as a raw material or as an intermediate, it must first be hydrolyzed to form a preponderance of benzyl alcohol. In accordance with the present invention, this hydrolysis step is used with feedstocks containing at least 90 mole %, preferably 95 mole %, of benzyl acetate. Feedstocks containing up to 10 mole %, preferably up to 5 mole %, benzaldehyde may also be used, since benzaldehyde does not interfere with the hydrolysis step and it is beneficially fed to the homologation step. Generally, in the case of the feed to the hydrolysis reaction, not more than 10% benzaldehyde is present. On a molar basis, the ratio of water to benzyl alcohol in the hydrolysis reaction is from 1:1 to 20:1, preferably from 2:1 to 8:1. The hydrolysis is catalyzed by acetic acid in accordance with a preferred embodiment of the invention. Acetic acid is readily available as a co-product from the homologation reaction, as will be described hereinafter, and is also a co-product from the hydrolysis. The use of acetic acid as the catalyst is particularly preferred because it does not require the addition of another component to the system. Catalyst concentrations of 0.005 to 0.5 mole equivalents per mole of benzyl acetate are generally used to achieve the desired reaction rate. Catalyst addition is most important at temperatures below 180°C.; otherwise, the reaction is sluggish. Temperatures for the hydrolysis are from 80 to 250°C., preferably from 130 to 220°C. Lower temperatures slow the rate of reaction, while higher temperatures lead to product decomposition. Pressure is not critical, though of course it must be sufficient to ensure that the reaction mixture is in the liquid phase. Depending on temperature, the pressure may vary from $9.8 \times 10^4$ Pa to $3.9 \times 10^6$ Pa (1 atm. to about 40 atm.), and preferably from $4.9 \times 10^5$ to $2.4 \times 10^6$ Pa (5 atm. to about 25 atm.). The time for the mixture to reach equilibrium depends on the catalyst concentration, temperature, and the starting mixture. Normally, times of from 0.5 to 5 hours are sufficient.

During the hydrolysis reaction, the composition approaches an equilibrium to form a product containing from 50 to 97 mole % of benzyl alcohol, preferably from 70 to 90 mole %, based on total benzyl compounds in the product. Unconverted benzyl acetate, from about 3 to 50 mole %, preferably from 10 to 30 mole %, based on total benzyl compounds in the product, remains in the effluent. Up to 10 mole %, preferably up to 5 mole %, based on total benzyl compounds in the product, of the benzaldehyde which was present in the hydrolysis feed may be fed directly to the homologation step. Neither the benzaldehyde nor the unconverted benzyl acetate must first be removed. The only separation necessary is the separation of the organic phase from the water phase. The water phase contains the bulk of the acetic acid plus small amounts of the three benzyl constituents. The organic phase containing the three benzyl constituents plus small amounts of acetic acid and water is fed directly to the homologation. These two phases may be separated by any conventional phase separating equipment, as is well known to those skilled in the art.

In the overall process in converting toluene to β-phenylethyl alcohol, the toluene may be initially acetoxylated to form benzyl acetate which, in turn, may be used as a feed to the hydrolysis or homologation steps heretofore described. In such an acetoxylation reaction, the toluene is reacted with acetic acid and molecular oxygen. The acetic acid may be conveniently obtained as a by-product from the hydrolysis of the benzyl acetate discussed above.

The acetic acid used in the acetoxylation step must be concentrated. Where the acetoxylation is performed in the liquid stage, it is preferred that 98% acetic acid be used, while in the vapor phase reaction a concentration of 95% is sufficient. Acetic acid-water mixtures, such as obtained from the hydrolysis step, may be concentrated to 95% by simple distillation. Where higher purity is required, this step may be followed by an azeotropic distillation as is well known in the art.

The acetoxylation of toluene in the vapor phase is shown in British Patent Specification 1,328,058 and in the liquid phase in *J. Org. Chem.*, Vol. 33, No. 11 (Nov. 1968) by Bryant et al., "A Palladium-Catalyzed Synthesis of Benzyl Esters from Methyl Benzenes." The former reference shows that the acetoxylation is carried out catalytically in the vapor phase at temperatures of from 100 to *150°C.*, preferably 150 to 200°C., and at pressures of from $9.8 \times 10^4$ to $1.1 \times 10^6$ Pa (1 to 11 atm.), preferably from $9.8 \times 10^4$ to $5.9 \times 10^5$ Pa (1 to 6 atm.). The catalyst which is used is palladium, preferably supported on a carrier. Co-catalysts such as bismuth and tin salts may also be used. Catalyst preparations are shown in the aforesaid references, as are various catalyst additives. The liquid phase process operates at temperatures of from 80 to 150°C., preferably at 100°C., and uses activated carbon as a support for the palladium.

During the course of the acetoxylation reaction, the gaseous reaction mixture is cooled to a temperature below 50°C., forming a liquid phase which consists essentially of benzyl acetate,

benzaldehyde, unreacted toluene and acetic acid. The toluene and acetic acid, along with any water formed during the reaction, may be separated from the liquid phase as the distillate in a simple distillation. The toluene may be phase separated from the water and the acetic acid concentrated to form glacial acetic acid for recycle as described above. The bottoms product from this distillation, containing 90 mole %, preferably 85 mole %, benzyl acetate, and up to 10 mole %, preferably not more than 5 mole %, benzaldehyde, is sent directly to the hydrolysis step.

In the combination of process steps of the present invention wherein benzaldehyde is converted to form a feedstock for the homologation reaction, the benzaldehyde is reduced in the presence of hydrogen and a reduction catalyst. As outlined in Rylander, "Catalytic Hydrogenation over Platinum Metals," Academic Press, New York, 1967, aromatic aldehydes are best reduced by palladium under mild conditions. Benzyl alcohol can be produced in high yield by reduction of benzaldehyde over palladium (5%) on carbon at 20 to 50°C. at 1 to 3 atm. pressure. Other noble metal catalysts can also be used. Acetic acid can be used as solvent. The reaction is stopped after the stoichiometric amount of hydrogen (1 equiv.) is added to ensure that hydrogenolysis to toluene does not take place. Conversion of benzaldehyde is 90 to 95%.

The effluent from the reduction contains at least 85 mole %, preferably at least 90 mole %, of benzyl alcohol (solvent-free basis) based on the total amount of benzyl compounds. In addition, in performing the reduction as recited previously, about 3 to 15 mole %, preferably 3 to 10 mole %, benzaldehyde, based on the total amount of benzyl compounds, remains unconverted. Because this material may be tolerated and in fact converted in the homologation reaction, it need not be removed by a finishing step.

The oxidation of toluene with molecular oxygen provides the first step of the second route from toluene to β-phenylethyl alcohol. In this scheme the toluene is partially oxidized to form benzaldehyde. The benzaldehyde is upgraded to a feedstock containing at least 95 mole % of benzaldehyde, preferably 98 mole %, and is thereafter fed to the above-described reaction step.

The oxidation to benzaldehyde is shown in German OLS 2,136,779 and by Ai in *Kogyo Kagaku Zasshi* (1971), *74,* 1636. In the German OLS, toluene in a vapor phase reaction is fed with excess air and a little steam over a mixed molybdenum oxide-iron oxide catalyst at 370°C. with a contact time of 2.1 seconds to give 69% benzaldehyde selectivity at 14.6% toluene conversion. In *Kogyo Kagaku Zasshi* (1971), *74,* 1636, toluene is fed in a vapor phase reaction over a mixed molybdenum oxide-phosphorous oxide catalyst at 550° and short contact time to produce benzaldehyde in 60% selectivity and benzaldehyde benzyl alcohol mixture in 75% selectivity.

Other oxidation catalysts may also be used in the oxidation of toluene. Examples of such catalysts are cerium molybdate, vanadium pentoxide and potassium sulfate on silica gel, uranium oxide, or molybdenum oxide.

The second embodiment of present invention relates to the preparation of β-phenylethyl alcohol and β-phenylethyl acetate by the homologation of benzyl alcohol. More specifically, the invention relates to such process wherein the dibenzyl ethers formed in the reaction are separated from the reactor effluent and recycled to the homologation reaction. β-Phenylethyl alcohol is a known article of commerce used in the preparation of organic chemicals in fragrance materials. It and β-phenylethyl acetate are also valuable intermediates in the preparation of styrene.

In the homologation of benzyl alcohol to produce β-phenylethyl alcohol, according to the present invention, the reactor effluent contains a blend of several components. In order to obtain high purity β-phenylethyl alcohol and β-phenylethyl acetate, the reactor effluent is initially settled and separated into an organic phase and a water phase. The organic phase, containing the desired products, is washed with aqueous acid, normally acetic acid, to extract a portion of the catalyst in the organic phase and thereafter subjected to a first distillation. In this first distillation, the lowest boiling materials, water, acetic acid, and toluene, are first separated as the distillates, followed by unreacted benzyl alcohol and benzyl acetate. The benzyl alcohol and benzyl acetate may be recycled to the homologation reactor and blended with fresh feed. The β-phenylethyl alcohol and β-phenylethyl acetate are thereafter separated as a distillate in a second distillation column. The residue of the second column contains, among other things, high boiling dibenzyl ethers and, in certain instances, components of the catalyst system. In the process of the present invention, the dibenzyl ethers, contained in the residue of the second column, are recycled to the homologation. Components of the catalyst system, contained in the residue of the second column, may also be recycled to the homologation.

The term "dibenzyl ether" as used herein refers not only to the specific dibenzyl ether compound, but also to higher molecular weight materials such as di-β-phenylethyl ether and benzyl-β-phenyl ether and similar higher boiling compounds. These materials may be represented by the following formula:

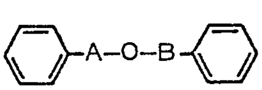

wherein A and B may be an alkylene group having 1 or 2 carbon atoms.

In the homologation reaction of this embodiment of the present invention, the feedstock must

contain at least 25 mole % benzyl alcohol, preferably at least 50 mole %, based on the total amount of benzyl alcohol, benzyl acetate, and benzaldehyde (benzyl compounds). From 3 to 15 mole % of benzaldehyde, preferably 3 to 10 mole %, and from 5 to 75 mole % of benzyl acetate, preferably from 5 to 50 mole %, may also be present in the feedstock in accordance with the first embodiment of the invention. The other condition of the homologation are as described above.

The dibenzyl ethers produced during the preparation of β-phenylethyl alcohol and β-phenylethyl acetate were generally regarded as a wasteful by-product because they were not capable of being converted directly to styrene or homologated to form β-phenylethyl alcohol or β-phenylethyl acetate. Generally, the feedstock to the reactor, after addition of the dibenzyl ether residue, will contain about 0.01 to 0.12 moles of the dibenzyl ether per mole of benzyl compounds, generally from 0.02 to 0.08. Under ideal equilibrium conditions there is no net production of heavy ethers. At equilibrium the ether level in the reaction product will range from 3 to 6 mole % of the benzyl compounds. Preferably all of these are recycled, though, of course, recycling even a part of these will be beneficial.

The dibenzyl ethers recovered from the residue of the second distillation may also contain other high boiling compounds, such as catalyst residue. Since the catalyst may also be usefully reemployed in the process, it is not essential to separate the other components from the residue in those cases wherein portions of the catalyst and the entire residue from the second distillation may be recycled.

The following examples illustrate first embodiment of the present invention more fully:

## Example 1

A 500 cc. Hastelloy (Trade Mark) C autoclave is charged with 189 grams of feed. Unless noted otherwise in the table below, this feed is composed of 165.6 grams of benzyl compounds, 14 grams of water, 7 grams of cobalt octacarbonyl, 0.9 grams of ruthenium trichloride hydrate and 1.5 grams of sodium iodide.

The autoclave is pressurized to 275 atm. with synthesis gas in a molar ratio of $H_2/CO$ of 1/1 and heated to 130°C. During a reaction period of 4 hours the synthesis gas is fed at a rate of 3 liters per minute (measured at 25°C. and $9.8 \times 10^4$ Pa (1 atmosphere)). After 4 hours, the autoclave is cooled to room temperature and depressurized. The liquid product is analyzed by gas-liquid chromatography.

A series of six runs are performed and are outlined in Table I. The first three are control runs performed with the individual benzyl compounds. Runs 4 and 5 demonstrate the effect of running binary mixtures with benzyl alcohol. Run 6 is performed with a ternary mixture of benzyl alcohol, benzyl acetate and benzaldehyde.

TABLE I

| Run No. | Benzyl Compounds — mole % | Comments |
|---|---|---|
| 1 | Benzyl Alcohol — 100 | — |
| 2 | Benzyl Acetate — 100 | 10.3% of the benzyl acetate was hydrolyzed to the alcohol. |
| 3 | Benzaldehyde — 100 | 30% of the benzaldehyde was reduced to the alcohol. |
| 4 | Benzyl Alcohol — 90 | — |
| | Benzaldehyde — 10 | |
| 5 | Benzyl Alcohol — 60 | — |
| | Benzyl Acetate — 40 | — |
| 6 | Benzyl Alcohol — 46 | — |
| | Benzyl Acetate — 47 | |
| | Benzaldehyde — 7 | |

TABLE I (continued)

| Run No. | % Conversion of Benzyl Alcohol | Benzyl Acetate | Benz-aldehyde |
|---|---|---|---|
| 1 | 39.5 | — | — |
| 2 | — | 15.5 | — |
| 3 | — | — | 99.4 |
| 4 | 46.2 | — | 98.7 |
| 5 | 28.1 | 46.6 | — |
| 6 | 27 | 41.3 | 99.4 |

| Run No. | % Molar Selectivity to β-Phenylethyl Alcohol & Acetate | Toluene | Benzyl Ethers | Benzyl Alcohol |
|---|---|---|---|---|
| 1 | 73.1 | 16.7 | 10.2 | 0 |
| 2 | 26.0 | 6.3 | 1.3 | 66.4 |
| 3 | 10.1 | 2.4 | 57.2 | 30.3 |
| 4 | 70.1 | 16.5 | 13.4 | 0 |
| 5 | 73.7 | 19.2 | 6.6 | 0 |
| 6 | 73 | 17.2 | 9.8 | 0 |

Analysis of the products obtained in Runs 4, 5 and 6 shows unexpectedly high conversions of the benzyl acetate and surprising selectivities to the β-phenylethyl alcohol and β-phenylethyl acetate from the benzaldehyde. A comparison of the predicted values, based on Runs 1 to 3, and the actual values obtained in Runs 4 to 6 is shown in Table II.

TABLE II

| | Linearized Predictions | | Actual Results | |
|---|---|---|---|---|
| Run No. | Conversion of all Benzyl Compounds | Molar Selectivity to β-phenylethyl Alcohol and Acetate | Conversion of all Benzyl Compounds | Molar Selectivity to β-phenylethyl Alcohol and Acetate |
| 4 | 36.7% | 63.6 | 46.2% | 70.1 |
| 5 | 25.4 | 73.4 | 35.9 | 73.7 |
| 6 | 30.6 | 57.6 | 40.8 | 73.0 |

The above table clearly shows the synergistic effect of reacting the benzaldehyde and benzyl acetate in the presence of benzyl alcohol.

Example 2

This example shows the embodiment of the invention employing acetoxylation, hydrolysis and homologation.

The following mixture is charged to a 500 cc. stainless steel autoclave:

| | |
|---|---|
| Toluene | 101.61 grams |
| Acetic acid | 132.47 grams |
| Potassium acetate | 27.13 grams |
| Tin oxide (SnO) | 9.12 grams |
| Palladium acetate $Pd(OAc)_2$ | 3.99 grams |
| Carbon | 14.45 grams |
| Acetic anhydride | .47 grams |
| | 289.24 |

The autoclave is sealed, pressurized to $2 \times 10^6$ Pa (20 atm.) with nitrogen, and heated to 133°C. Air is fed at an average rate of 556 cc/min. (measured at atmospheric pressure and 25°C.), for three hours. After three hours, the autoclave is cooled and depressurized. The recovered products weight 283.9 grams (or 98.2% of the initial charge). The reactor effluent is filtered to recover the solids. The liquids analysis, by gas chromatograph, is as follows:

| Product | Wt. % |
|---|---|
| Toluene | 16.8 |
| Acetic acid | 40.07 |
| Benzaldehyde | 1.91 |
| Benzyl acetate | 26.13 |
| Water | 3.63 |
| Potassium acetate | 10.37 |
| Others (Benzylidene diacetate and others) | 1.09 |
| Acetic anhydride | |
| | 100.00 |

Calculation shows a conversion of toluene of 46.8% and molar selectivities of 88.2% to benzyl acetate, 9.1% to benzaldehyde and 1.9% to benzylidene diacetate. This product is distilled to first remove the water, acetic acid and toluene. The remaining product is water-washed at room temperature to remove the acetate salts and further distilled to produce 65 grams of product composed of 7 wt. % benzaldehyde and 93 wt. % benzyl acetate. The total product is distilled overhead and the diacetate remains as the residue. The remaining materials can be recycled to the oxidation step.

This benzyl acetate product from the acetoxylation step is partially hydrolyzed as follows. Water and acetic acid are added to the product in the ratios of 0.5 g./g. and 0.015 g./g., respectively. The mixture is heated to 180°C. in a glass pressure bottle under a nitrogen atmosphere. The system pressure is $1.5 \times 10^6$ Pa (15 atm.). These conditions are maintained for 5 hours. Thereafter, the bottle is cooled to room temperature and two phases form. The water phase contains 9 wt. % organics and 20 wt. % acetic acid which can be reused in another hydrolysis. The organic phase accounts for 79 wt. % of the product and has the following composition:

| Component | Wt. % |
|---|---|
| Benzaldehyde | 5 |
| Benzyl alcohol | 38 |
| Benzyl acetate | 28 |
| Water | 12 |
| Acetic acid | 17 |

This organic phase is distilled to remove the water and acetic acid, leaving an organic product composed of 7 wt. % (8.1 mole %) benzaldehyde, 53.5 wt. % (61.3 mole %) benzyl alcohol, and 39.5 wt. % (30.1 mole %) benzyl acetate.

This product is fed to a homologation reaction in accordance with the procedure described in Run 6 of Example 1. In this step the benzaldehyde conversion is 99%, the benzyl acetate conversion is 38%, and the benzyl alcohol conversion is 30%. The molar selectivity of β-phenylethyl alcohol plus acetate based upon converted benzyl compounds is 74%.

Example 3

In this example the distilled but unhydrolyzed acetoxylation product of Example 2 containing 7 wt. % benzaldehyde and 93 wt. % benzyl acetate is joined with the unreacted homologation feed from Example 2 in the ratio of 1 g. to 1.5 g., respectively to form the following feedstock:

| Compound | Acetoxylation Feed, wt. % | Homologation Recycle-wt. % | New Homologation Feed, wt. % |
|---|---|---|---|
| Benzaldehyde | 7 (10.2 mole %) | 0.1 | 2.9 |
| Benzyl Acetate | 93 (89.8 mole %) | 42.3 | 62.6 |
| Benzyl Alcohol | 0 (0 mole %) | 57.6 | 34.5 |

This is fed to a homologation reaction with equipment and conditions as described in Run 6 of Example 1. In this step the benzaldehyde conversion is 99%, the benzyl acetate conversion is 50%, and the benzyl alcohol conversion is 20%. The molar selectivity of β-phenylethyl alcohol plus acetate based upon converted benzyl compounds is 71%.

Example 4

A toluene oxidation catalyst is prepared by mixing 2500 ml. $H_2O$ and 2500 ml. concentrated $NH_3$ and adding to this 500 g. $UO_3$, 48.4 g. $MoO_3$, 90 ml. $H_2SO_4$, 5.5 g. $KNO_3$ and 840.5 g. citric acid. The mixture is refluxed for 4 hours, dried and calcined in air at 600°C. for 4 hours. Granules of this catalyst are placed in a tubular reactor and contacted with a mixture of toluene, steam, and air in the molar ratio of 1.5 to 10 to 88.5 at 400°C. and $9.8 \times 10^4$ Pa (1 atm.) pressure with a residence time of 2 seconds. The conversion of toluene is 27% and the selectivity to benzaldehyde was 76 mole %. The benzaldehyde is readily separated from the condensed liquids by distillation.

100 g. of benzaldehyde are mixed with 300 g. of acetic acid and 5 g. of 5 wt. % palladium on carbon. The slurry is sparged with nitrogen, brought to 40°C., and then pressured to $2.9 \times 10^5$ Pa (3 atm.) with hydrogen. Hydrogen uptake is monitored and the reaction is stopped before complete conversion occurs. Conversion of benzaldehyde is 88% and selectivity to benzyl alcohol is 98%. Filtration and distillation of the reaction liquid lead to a product containing 10 wt. % (10 mole %) benzaldehyde and 90 wt. % (90 mole %) benzyl alcohol.

This product is homologated to produce β-phenylethyl alcohol as described in Example 1, Run 4. Similar results are obtained.

The following examples demonstrate the second embodiment of the present invention more fully:

Example 5

In this example, five runs are performed in a 500 cc. Hastelloy autoclave pressurized at $2.7 \times 10^7$ Pa (275 atm.) with a synthesis gas containing equal moles of hydrogen and carbon monoxide. In each instance, the autoclave is heated to 130°C. and maintained at such temperature for a period of 4 hours while the synthesis gas is fed at the rate of 4 liters per minute (measured at 25°C. at $9.8 \times 10^4$ Pa (1 atm.) pressure). After the reaction period, the autoclave is cooled to room temperature, depressurized, and the product recovered and analyzed.

In Run 1, the organic components of the feedstock contained essentially only the benzyl alcohol. In Runs 2 through 5, the dibenzyl ether recovered from the product of the previous run is added to the feedstock, and sufficient benzyl alcohol added so that the total organic feed is approximately 165 g. In each case the $Co_2(CO)_8$ catalyst was promoted with 0.9 g. of $RuCl_3 \cdot H_2O$ (38.1% Ru) and 1.5 g. of sodium iodide. The other components of the feed to each run are shown in Table III below.

TABLE III

| Run # | 1 | 2 | 3 | 4 | 5 |
|---|---|---|---|---|---|
| *Feed* | | | | | |
| Benzyl alcohol, g. | 165.6 | 160.0 | 155.0 | 155.0 | 154.0 |
| Heavy ethers, g. | 0 | 6.0 | 9.2 | 10.7 | 10.9 |
| Water, g. | 13.8 | 13.9 | 13.6 | 13.7 | 13.8 |
| $Co_2(CO)_8$, g. | 7.0 | 7.0 | 7.2 | 7.1 | 7.1 |
| *Product Analysis* | | | | | |
| Benzyl alcohol conversion, % | 41 | 43 | 45 | 44 | 45 |
| Molar selectivity to: | | | | | |
| β-phenylethyl alcohol, % | 73.1 | 78.7 | 80.8 | 82.5 | 83.0 |
| Toluene, % | 16.7 | 16.3 | 17.0 | 17.1 | 17.0 |
| Heavy ethers, % | 10.2 | 5.0 | 2.2 | 0.4 | negligible |

The foregoing table shows the percent conversion based on benzyl alcohol as well as the molar selectivity to β-phenylethyl alcohol, toluene, and dibenzyl ethers. The products are separated by a combination of extraction and fractionation.

As will be observed from the product analysis, the net production of the heavy ethers decreases as the amount of heavy ethers in the feedstock is increased. At equilibrium, the net production of the heavy ethers is nil. Surprisingly, the molar selectivity to the β-phenylethyl alcohol increases markedly even at the higher conversions achieved.

Example 6

This example shows the application of the invention to a homologation feedstock containing benzyl alcohol in admixture with benzyl acetate and benzaldehyde. The same procedure is followed as in Example 5, except that the molar ratio of $H_2/CO$ is 2/1 and the gas feed rate is 2 liters/minute. Table IV below shows the product analysis and the molar selectivity.

TABLE IV

| Run # | 6 | | 7 | |
|---|---|---|---|---|
| *Feed* | Grams | % by Wt. | Grams | % by Wt. |
| Benzyl alcohol | 75.1 | 38.87 | 68.1 | 35.37 |
| Benzyl acetate | 75.1 | 38.87 | 68.1 | 35.37 |
| Benzaldehyde | 16.5 | 8.54 | 16.6 | 8.62 |
| Heavy ethers* | 0.0 | 0.0 | 13.2 | 6.85 |
| Water | 14.0 | 7.24 | 14.0 | 7.27 |
| $Co(OAc)_2 \cdot 4H_2O$ | 10.1 | 5.23 | 10.2 | 5.28 |
| $RuCl_3 \cdot nH_2O$ (38.1% Ru) | 0.9 | 0.46 | 0.9 | 0.46 |
| NaI | 1.5 | 0.78 | 1.5 | 0.78 |
| Total | 193.2 | 100.00 | 192.6 | 100.00 |

* Heavy bottoms from previous runs.

TABLE IV (continued)

| *Product Analysis* | | |
|---|---|---|
| Percent Conversion of | % by wt. | % by wt. |
| Benzyl alcohol | 20.1 | 22.1 |
| Benzyl acetate | 40.0 | 41.3 |
| Benzaldehyde | 99.2 | 99.4 |
| All benzyl compounds (molar) | 36.5 | 38.8 |
| Molar Selectivity to | | |
| β-phenylethyl alcohol acetate | 73.9 | 81.2 |
| Toluene | 16.0 | 15.2 |
| Heavy ethers | 10.1 | 3.6 |

In these examples, the heavy ether from Run 6 is included in the feed to Run 7 and the other benzyl components reduced proportionately.

This example illustrates that the recycle of the heavy ethers to the homologation reaction is also effective when the feed contains benzaldehyde and benzyl acetate. Though the level of heavy ethers in the feed to Run 7 is not sufficient to completely suppress the formation of additional heavy ethers, it can be seen that the selectivity to the β-phenylethyl alcohol and the β-phenylethyl acetate is increased by 7.3%. It would be expected that this selectivity increase would be even greater at steady state conditions.

**Claims**

1. A process for the preparation of β-phenylethyl alcohol and β-phenylethyl acetate by the homologation of benzyl alcohol with carbon monoxide and hydrogen in the presence of a cobalt catalyst, characterised in that the feed to the homologation reaction includes benzyl acetate, benzaldehyde or a dibenzyl ether or combination thereof, in addition to benzyl alcohol.

2. A process according to claim 1, characterised in that the feed to the homologation reaction contains (A) at least 25 mole % of benzyl alcohol and (B) from 5 to 75 mole % of benzyl acetate or from 3 to 15 mole % of benzaldehyde, said mole percentages being based on total benzyl compounds.

3. A process according to claim 1 or claim 2, characterised in that the cobalt catalyst is promoted with a ruthenium source and an iodide source.

4. A process according to any of the preceding claims, characterised in that the cobalt catalyst is promoted with a ruthenium source and an iodide source in the presence of at least 0.1 weight percent water (based on the feedstock) at a temperature of at least $6.8 \times 10^6$ Pa (70 atmospheres) and at a temperature of from 100 to 165°C.

5. A process according to any of the preceding claims, characterised in that the feedstock is prepared by hydrolyzing benzyl acetate in the presence of an acid catalyst; separating the organic phase and the water phase formed in the hydrolysis effluent, said organic phase containing predominantly benzyl alcohol and benzyl acetate; and using at least the benzyl compounds in said organic phase as the feedstock to the homologation.

6. A process according to claim 5 characterised in that the organic phase contains at least 25 mole % benzyl alcohol and from 3 to 75 mole % benzyl acetate, based on total benzyl compounds.

7. A process according to any of claims 1 to 5, characterised in that the feedstock is prepared by: acetoxylating toluene with molecular oxygen in the presence of acetic acid to form a reaction product containing predominantly benzyl acetate and minor amounts of benzaldehyde; passing said reaction product to a hydrolysis step in the presence of an acetic acid catalyst so as to form an organic phase containing at least 25 mole % benzyl alcohol and from 3 to 75 mole % benzyl acetate, based on total benzyl compounds, and an aqueous phase; separating said organic phase from said aqueous phase; and using at least the benzyl compounds in said organic phase as the feedstock to the homologation.

8. A process according to claim 7, characterised in that the aqueous phase from said hydrolysis contains water and acetic acid and wherein at least part of the acetic acid is separated from the water and recycled to said acetoxylation step.

9. A process according to claim 1, characterised in that the feedstock is prepared by: reducing benzaldehyde by reaction with hydrogen in the presence of a reduction catalyst to form a reactor effluent containing benzyl alcohol and from 3 to 15 mole % of benzaldehyde, based on total benzyl

compounds; and directly using said reactor effluent as the feedstock to the homologation.

10. A process according to claim 1, characterised in that the feedstock is prepared by: oxidizing toluene with molecular oxygen in the presence of an oxidation catalyst, thereby forming a first reactor effluent containing benzaldehyde; passing said benzaldehyde to a reduction zone; reducing said benzaldehyde in the presence of hydrogen and a reduction catalyst, thereby forming a second reaction effluent containing benzyl alcohol and residual benzaldehyde; thereafter using said second reaction effluent as the feedstock to the homologation.

11. A process according to any of the preceding claims, characterised in that from 0.01 to 0.12 mole of one or more dibenzyl ethers is present in the feed to the homologation reaction for each mole of the benzyl compounds.

12. A process according to any of the preceding claims, characterised in that the β-phenylethyl alcohol and high boiling components containing dibenzyl ethers are removed from the homologation reactor effluent, the β-phenylethyl alcohol is separated as a distillate from the high boiling components, and the dibenzyl ethers present in the high boiling components are recycled as part of the feedstock to the homologation.

13. A process according to claim 11 or claim 12, characterised in that the dibenzyl ethers are dibenzyl ether, di-β-phenylethyl ether and benzyl-β-phenyl ether.

**Revendications**

1. Procédé de préparation d'alcool de β-phényléthyle et d'acétate de β-phényléthyle par l'homologation d'alcool benzylique avec de l'oxyde de carbone et de l'hydrogène en présence d'un catalyseur de cobalt, caractérisé en ce que l'alimentation vers la réaction d'homologation contient de l'acétate de benzyle, du benzaldéhyde ou un dibenzyl éther ou leur combinaison, en plus de l'alcool benzylique.

2. Procédé selon la revendication 1, caractérisé en ce que l'alimentation vers la réaction d'homologation contient (A) au moins 25 mole % d'alcool benzylique et (B) 5 à 75 mole % d'acétate de benzyle ou de 3 à 15 mole % de benzaldéhyde, lesdits pourcentages en mole étant basés sur les composés totaux de benzyle.

3. Procédé selon la revendication 1 ou la revendication 2, caractérisé en ce que le catalyseur de cobalt est favorisé avec une source de ruthénium et une source d'iodure.

4. Procédé selon l'une quelconque des revendications précédentes, caractérisé en ce que le catalyseur de cobalt est favorisé avec une source de ruthénium et une source d'iodure en présence d'au moins 0,1% en poids d'eau (en se basant sur l'alimentation) à une pression d'au moins $6,8 \times 10^6$ Pa et à une température de 100 à 165°C.

5. Procédé selon l'une quelconque des revendications précédentes, caractérisé en ce que l'alimentation est préparée en hydrolysant de l'acétate de benzyle en présence d'un catalyseur acide; en séparant la phase organique et la phase d'eau qui sont formées dans l'effluent de l'hydrolyse, ladite phase organique contenant de façon prédominante de l'alcool benzylique et de l'acétate de benzyle; et en utilisant au moins les composés de benzyle dans ladite phase organique comme produit d'alimentation pour l'homologation.

6. Procédé selon la revendication 5, caractérisé en ce que la phase organique contient au moins 25 mole % d'alcool benzylique et de 3 à 75 mole % d'acétate de benzyle, en se basant sur les composés totaux de benzyle.

7. Procédé selon l'une des revendications 1 à 5, caractérisé en ce que l'alimentation est préparée en acétoxylant du toluène avec de l'oxygène moléculaire en présence d'acide acétique pour former un produit réactionnel contenant de façon prédominante de l'acétate de benzyle et des quantités mineures de benzaldéhyde; en faisant passer ledit produit réactionnel vers une étape d'hydrolyse en présence d'un catalyseur d'acide acétique afin de former une phase organique contenant au moins 25 mole % d'alcool benzylique et de 3 à 75 mole % d'acétate de benzyle, en se basant sur les composés totaux de benzyle, et une phase aqueuse; en séparant ladite phase organique de ladite phase aqueuse; et en utilisant au moins les composés de benzyle dans ladite phase organique comme produit d'alimentation vers l'homologation.

8. Procédé selon la revendication 7, caractérisé en ce que la phase aqueuse de l'hydrolyse contient de l'eau et de l'acide acétique et en ce qu'au moins une partie d'acide acétique est séparée de l'eau et est recyclée vers ladite étape d'acétoxylation.

9. Procédé selon la revendication 1, caractérisé en ce que le produit d'alimentation est préparé en réduisant du benzaldéhyde par réaction avec de l'hydrogène en présence d'un catalyseur de réduction pour former un effluent du réacteur contenant de l'alcool benzylique et de 3 à 15 mole % de benzaldéhyde, en se basant sur les composés totaux de benzyle; et en utilisant directement ledit effluent du réacteur comme produit d'alimentation vers l'homologation.

10. Procédé selon la revendication 1, caractérisé en ce que le produit d'alimentation est préparé en oxydant du toluène avec de l'oxygène moléculaire en présence d'un catalyseur d'oxydation, afin de former ainsi un premier effluent du réacteur contenant du benzaldéhyde; en faisant passer ledit

benzaldéhyde vers une zone de réduction; en réduisant ledit benzaldéhyde en présence d'hydrogène et d'un catalyseur de réduction, afin de former ainsi un second effluent de réaction contenant de l'alcool benzylique et du benzaldéhyde résiduel; et en utilisant ensuite ledit second effluent de réaction comme produit d'alimentation vers l'homologation.

11. Procédé selon l'une des revendications précédentes, caractérisé en ce qu'il y a de 0,01 à 0,12 mole d'un ou plusieurs dibenzyl éthers dans l'alimentation vers la réaction d'homologation pour chaque mole des composés de benzyle.

12. Procédé selon l'une quelconque des revendications précédentes, caractérisé en ce que l'alcool de $\beta$-phényléthyle et les composants à fort point d'ébullition contenant du dibenzyl éther sont retirés de l'effluent du réacteur d'homologation, l'alcool de $\beta$-phényléthyle étant séparé comme distillat des composants à fort point d'ébullition et les dibenzyl éthers présents dans les composants à fort point d'ébullition sont recyclés en tant que partie du produit d'alimentation vers l'homologation.

13. Procédé selon la revendication 11 ou la revendication 12, caractérisé en ce que les dibenzyl éthers sont du dibenzyl éther, du di-$\beta$-phényléthyl éther et du benzyl-$\beta$-phényl éther.

**Patentansprüche**

1. Verfahren zur Herstellung von $\beta$-Phenyläthylalkohol und $\beta$-Phenyläthylacetat durch Homologisierungsreaktion von Benzylalkohol mit Kohlenmonoxid und Wasserstoff im Gegenwart eines Kobaltkatalysators, dadurch gekennzeichnet, daß die Zufuhr für die Homologisierungsreaktion Benzylacetat, Benzaldehyd oder einen Dibenzyläther oder eine Kombination davon, zusätzlich zum Benzylalkohol, enthält.

2. Verfahren nach Anspruch 1, dadurch gekennzeichnet, daß die Zufuhr für die Homologisierungsreaktion (A) wenigstens 25 Mol % Benzylalkohol und (B) 5 bis 75 Mol % Benzylacetat oder 3 bis 15 Mol % Benzaldehyd enthält, wobei die Molprozentangaben auf die gesamten Benzylverbindungen bezogen sind.

3. Verfahren nach Anspruch 1 oder 2, dadurch gekennzeichnet, daß der Kobaltkatalysator mit einer Rutheniumquelle oder einer Iodidquelle aktiviert wird.

4. Verfahren nach einem der vorhergehenden Ansprüche, dadurch gekennzeichnet, daß der Kobaltkatalysator mit einer Rutheniumquelle und einer Iodidquelle in Gegenwart von wenigstens 0,1 Gew.% Wasser (bezogen auf das Einsatzmaterial) bei einem Druck von wenigstens $6{,}8 \times 10^6$ Pa und bei einer Temperatur von 100 bis 165°C aktiviert wird.

5. Verfahren nach einem der vorhergehenden Ansprüche, dadurch gekennzeichnet, daß das Einsatzmaterial hergestellt wird, indem man Benzylacetat in Gegenwart eines sauren Katalysators hydrolisiert, die organische Phase und die in dem Hydrolysenausstrom gebildete Wasser phase abtrennt, wobei die organische Phase hauptsächlich Benzylalkohol und Benzylacetat enthält und wenigstens die Benzylverbindungen in der organischen Phase als Einsatzmaterial für die Homologisierungsreaktion verwendet.

6. Verfahren nach Anspruch 5, dadurch gekennzeichnet, daß die organische Phase wenigstens 25 Mol % Benzylalkohol und 3 bis 75 Mol % Benzylacetat, bezogen auf die gesamten Benzylverbindungen enthält.

7. Verfahren nach einem der Ansprüche 1 bis 5, dadurch gekennzeichnet, daß das Einsatzmaterial hergestellt wird, indem man Toluol mit molekularem Sauerstoff in Gegenwart von Essigsäure zur Bildung eines Reaktions-produktes acetoxyliert, das überwiegend Benzylacetat und kleinere Mengen Benzaldehyd enthält, das Reaktions-produkt einer Hydrolysenstufe in Gegenwart von Essigsäure als Katalysator unterwirft, so daß eine organische Phase mit einem Gehalt von wenigstens 25 Mol % Benzylalkohol und 3 bis 75 Mol % Benzylacetat, bezogen auf die gesamten Benzylverbindungen und eine wäßrige Phase gebildet werden, die organische Phase von der wäßrigen Phase abtrennt und wenigstens die Benzylverbindungen in der organischen Phase als Einsatzmaterial für die Homologisierungsreaktion verwendet.

8. Verfahren nach Anspruch 7, dadurch gekennzeichnet, daß die wäßrige Phase aus der Hydrolysenstrufe Wasser und Essigsäure enthält, und wobei wenigstens ein Teil der Essigsäure von dem Wasser abgetrennt und zu der Acetoxylierungsstufe im Kreislauf zurückgeführt wird.

9. Verfahren nach Anspruch 1, dadurch gekennzeichnet, daß das Einsatzmaterial hergestellt wird, indem man Benzaldehyd durch Umsetzung mit Wasserstoff in Gegenwart eines Reduktionskatalysators unter Bildung eines aus dem Reaktor ausfließenden Stroms mit einem Gehalt von Benzylalkohol und 3 bis 15 Mol % Benzaldehyd, bezogen auf die gesamten Benzylverbindungen, reduziert und den aus dem Reaktor ausfließenden Strom als Einsatzmaterial für die Homologisierungsreaktion unmittelbar verwendet.

10. Verfahren nach Anspruch 1, dadurch gekennzeichnet, daß das Einsatzmaterial hergestellt wird, indem man Toluol mit molekularem Sauerstoff in Gegenwart eines Oxidationskatalysators oxidiert, wodurch ein erster aus dem Reaktor ausfließender Strom mit einem Gehalt von Benzaldehyd gebildet wird, den Benzaldehyd zu einer Reduktionszone leitet, in der der Benzaldehyd in Gegenwart von Wasserstoff und einem Reduktionskatalysator reduziert wird, wodurch ein zweiter aus dem Reaktor ausfließender Strom mit einem Gehalt von Benzylalkohol und restlichem Benzaldehyd gebildet wird und

danach den zweiten aus dem Reaktor ausfließenden Strom als Einsatzmaterial für die Homologisierungsreaktion verwendet.

11. Verfahren nach einem der vorhergehenden Ansprüche, dadurch gekennzeichnet, daß ein oder mehrere Dibenzyläther in einer Menge von 0,01 bis 0,12 Mol pro Mol der Benzylverbindungen in der Zufuhr für die Homologisierungsreaktion vorhanden ist bzw. sind.

12. Verfahren nach einem der vorhergehenden Ansprüche, dadurch gekennzeichnet, daß der $\beta$-Phenyläthylalkohol und hochsiedende Komponenten mit einem Gehalt von Dibenzyläthern von dem Strom abgetrennt werden, der aus dem Homologisierungsreaktor ausfließt, der $\beta$-Phenyläthylalkohol als Destillat von den hochsiedenden Komponenten abgetrennt wird und die in den hochsiedenden Komponenten vorhandenen Dibenzyläther als Teil des Einsatzmaterials zu der Homologisierungsreaktion im Kreislauf zurückgeführt werden.

13. Verfahren nach Anspruch 11 oder 12, dadurch gekennzeichnet, daß Dibenzyläther, Di-$\beta$-Phenyläthyläther und Benzyl-$\beta$-phenyläthyläther als Dibenzyläther eingesetzt werden.